# EUROPÄISCHE PATENTSCHRIFT

(11) **EP 2 850 066 B1**
(45) Veröffentlichungstag und Bekanntmachung des Hinweises auf die Patenterteilung: **16.03.2016**
(21) Anmeldenummer: 13725092.4
(22) Anmeldetag: 10.05.2013
(51) Int. Cl.: C07D 231/14

(54) **VERFAHREN ZUM HERSTELLEN VON 1-ALKYL-3-FLUORALKYL-1H-PYRAZOL-4-CARBONSÄURECHLORIDEN**
METHOD FOR PRODUCING 1-ALKYL-3-FLUORALKYL-1H-PYRAZOL-CARBOXYLIC ACID CHLORIDES
PROCÉDÉ DE FABRICATION DE CHLORURES D'ACIDE 1-ALKYL-3-FLUORALKYL-1H-PYRAZOLE-4-CARBOXYLIQUE

(30) Priorität: 14.05.2012 EP 12167855
(43) Veröffentlichungstag der Anmeldung: 25.03.2015
(73) Patentinhaber: Bayer CropScience AG, 40789 Monheim am Rhein (DE)
(72) Erfinder: PAZENOK, Sergii, 42699 Solingen (DE); LUI, Norbert, 51519 Odenthal (DE); MÜLLER, Thomas Norbert, 40789 Monheim (DE)
(74) Vertreter: BIP Patents
(86) Internationale Anmeldenummer: PCT/EP2013/059745
(87) Internationale Veröffentlichungsnummer: WO 2013/171134

(56) Entgegenhaltungen:
- WO-A1-93/11117
- WO-A1-2004/063165
- WO-A2-2008/086962

## Beschreibung

Die vorliegende Erfindung betrifft ein neues Verfahren zum Herstellen von 1-alkyl-3-fluoralkyl-1H-pyrazol-4-carbonsäurechloriden, einer wertvollen Vorstufe für die Herstellung von Fungiziden, mittels reduktiver Dehalogenierung, ausgehend von N-Alkyl-3-haloalkyl-5-halo-pyrazolcarbaldehyd.

1-Alkyl-3-haloalkyl-Pyrazolcarbonsäurechloride sind wichtige Bausteine zur Herstellung von Pflanzenschutzwirkstoffen, insbesondere von SDHI-Fungiziden. Unsubstituierte Pyrazolcarbonsäurechloride stellt man üblicherweise durch Umsetzung von Carbonsäuren mit einem Chlorierungsmittel dar. Ein Vorteil dieses Verfahrens beruht darauf, dass die entsprechenden Carbonsäuren einfach zugänglich und damit im technischen Maßstab verfügbar sind. Diese Voraussetzung ist bei der Herstellung von substituierten Pyrazolcarbonsäurechloriden nicht gegeben, da die entsprechenden Carbonsäuren nicht leicht zugänglich sind. 1-Alkyl-3-haloalkyl-pyrazolcarbonsäurechloride werden üblicherweise in einer Mehrstufigen Transformation ausgehend von Fluoralkylacetoacetat hergestellt:

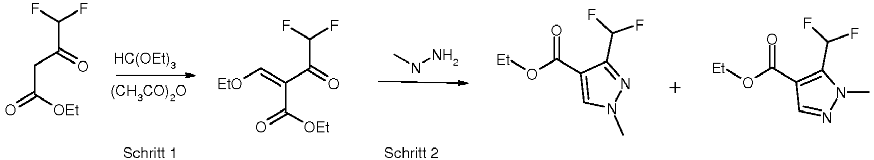

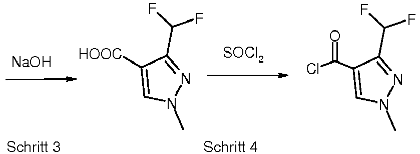

Insbesondere die Schritte 1 und 2 sind technisch anspruchsvoll. In Schritt 1 ist ein sehr großer Überschuss (2-3 Äquivalente) an Essigsäureanhydrid notwendig, um eine vollständige Transformation zu gewährleisten. Die Isolierung des Produkts von Schritt 1 und seine Trennung von dem Überschuss an Essigsäureanhydrid oder Essigsäure ist teuer und sehr zeitaufwändig. In Schritt 2 macht die Bildung des zweiten Isomers (bis zu 15 %) und dessen Abtrennung das Verfahren schwierig und teuer.

Es wurde nun gefunden, dass man 1-alkyl-3-fluoralkyl-1H-pyrazol-4-carbonsäurechloride der Formel (I) 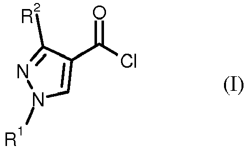 in welcher R¹ für C₁-C₆-Alkyl und R² für C₁-C₅-Fluoralkyl stehen, erhält, indem man

### 5-Halo-1-alkyl-3-fluoralkyl-1H-pyrazol-4-carbaldehyd der Formel (II)

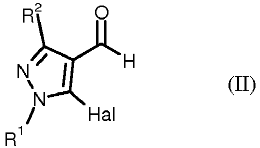

in welcher Hal für Cl, Br oder I steht und
R¹ und R² die oben angegebenen Bedeutungen haben,
in einem ersten Schritt mittels katalytischer Hydrierung und in Anwesenheit einer Base zu

### 1-alkyl-3-fluoralkyl-1H-pyrazol-4-carbaldehyd der Formel (III)

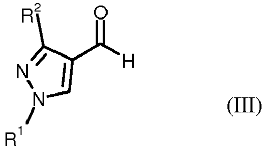

in welcher R¹ und R² die oben angegebenen Bedeutungen haben, umsetzt,
und dann die Verbindungen der Formel (III) durch Umsetzung mit einem Chlorierungsmittel unter Zusatz eines Radikalstarters (Schritt 2) in die Säurechloride der Formel (I) überführt.

Das erfindungsgemäße Verfahren kann durch das folgende Formelschema veranschaulicht werden:

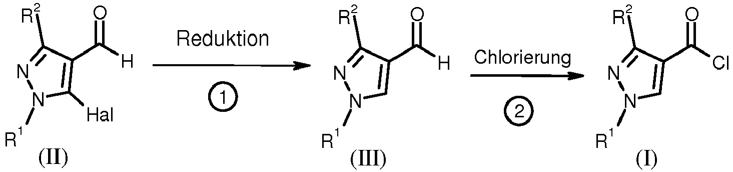

Die bei der Durchführung des erfindungsgemäßen Verfahrens als Ausgangsstoffe verwendeten 5-Halo-1-alkyl-3-fluoralkyl-1H-pyrazol-4-carbaldehyde sind durch die Formel (II) allgemein definiert. Der Rest R¹ steht in dieser Formel (II) bevorzugt für Methyl, Ethyl, n-Propyl, Isopropyl, Butyl, Pentyl, besonders bevorzug für Methyl. Der Rest R² steht für C₁-C₅-Fluoralkyl, worin Fluoralkyl eine Alkylgruppe mit 1 bis 5 Kohlenstoffatomen bedeutet, welche mit mindestens einem Fluoratom bis hin zur Perfluorierung substituiert ist. Wenn das Fluoralkyl nicht perfluoriert ist, können weitere Halogenatome wie Chlor und Brom, bevorzugt Chlor, als weitere Substituenten vorhanden sein. R² steht bevorzugt für CF₂H, CF₃, CF₂Cl, CCl₂F, C₂F₅, C₃F₇, besonders Bevorzugt für CF₂H und CF₃. Ganz besonders Bevorzugt setzt man 5-Chlor-1-methyl-3-difluormethyl-1H-pyrazol-4-carbaldehyd (II-1) als Ausgangsstoff ein.

Hal steht für Cl, Br oder I, bevorzugt für Cl oder Br, besonders bevorzugt für Cl.

In der WO 93/011117 A1 wird die Synthese einer 1-Methyl-3-trifluormethyl-4-pyrazol-carbonsäure offenbart, ausgehend von dem entsprechenden Carboxaldehyd. Die WO 93/011117 A1 offenbart jedoch nicht die erfindungsgemäße Synthese eines Pyrazol-Carbonsäurechlorids aus dem entsprechenden Carboxaldehyd. Auch der erfindungsgemäße Dehalogenierungsschritt mittels katalytischer Hydrierung ist in der WO 93/011117 A1 nicht offenbart.

Die WO 2008/086962 A2 offenbart ein Verfahren zum Herstellen von substituierten, in 5-Position halogenierten Pyrazolcarbonsäurechloriden aus dem entsprechenden Aldehyd. Der erfindungsgemäße Dehalogenierungsschritt mittels katalytischer Hydrierung ist auch in der WO 2008/086962 A2 nicht offenbart.

Die WO 2004/063165 A1 beschreibt die Entfernung eines Chloratoms in N-Aryl-3-methyl-5-chlorpyrazol carbaldehyden. Die Reduktion von 5-Halo-N-alkyl-3-haloalkyl-pyrazol-4-carbaldehyden ist im Stand der Technik nicht bekannt. Gleichzeitig ist bekannt, dass eine Haloalkylgruppe (CF₂Cl) in Position 3 von 1-substituiertem-Pyrazol-4-carboxylat partiell reduziert werden kann, wobei 1-substituierte 3-halo-3-fluoroalkyl-pyrazol-4-carbonsäuren gebildet werden (WO 2012/010692 A1).

Überraschenderweise wurde nun gefunden, dass es unter spezifischen Bedingungen möglich ist, selektiv ein Halogen Atom von N-Alkyl-3-haloalkyl-5-halopyrazolcarbaldehyd zu entfernen, ohne dabei die Haloalkyl gruppe in Position 3 zu beeinträchtigen oder zu reduzieren und auch ohne die Aldehydgruppe in Position 4 zu reduzieren.

5-Halo-1-alkyl-3-fluoralkyl-1H-pyrazol-4-carbaldehyde der Formel (II) sind bekannt oder lassen sich nach bekannten Verfahren herstellen (vgl. J. Het Chem. 1990, 27, 243, WO 2006/018725 A1, WO 2011/061205 A1).

### 5-Halo-1-alkyl-3-fluoralkyl-1H-pyrazol-4-carbaldehyde der Formel (II) lassen sich beispielsweise auf einfache Weise und günstige Weise herstellen, indem man

### (a) Ester der Formel (V)

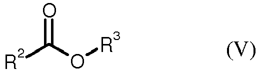

in welcher R² die oben angegebenen Bedeutungen hat und R³ für Methyl oder Ethyl steht,
mit Ethylacetat in Gegenwart einer Base (z.B. Natriumhydrid oder Na-Ethanolat) zu β-Ketoestern der Formel (VI) 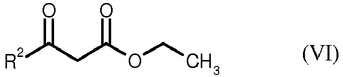 in welcher R² die oben angegebenen Bedeutungen hat, umsetzt, und diese

### (b) mit Alkylhydrazinen der Formel (VII)

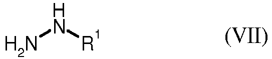

in welcher R¹ die oben angegebenen Bedeutungen hat,
in Gegenwart eines Verdünnungsmittels (z.B. Toluol) zu 5-Hydroxy-pyrazolen der Formel (VIII) 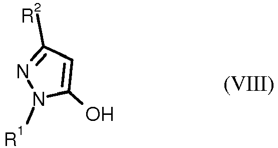 in welcher R¹ und R² die oben angegebenen Bedeutungen haben, umsetzt, und diese

(c) in einem letzten Schritt in Gegenwart mit einem Halogenierungsmittel (z.B. Phosphoroxychlorid, Phosphoroxybromid oder Phosphoroxyiodid), gegebenfalls in Gegenwart eines Verdünnungsmittels (z.B. Toluol), oder ohne ein Verdünnungsmittel, und in Gegenwart von Dimethylformamid, umsetzt.

Jeder Schritt dieses Verfahrens wird unter Verwendung günstiger Startmaterialien durchgeführt und ist vollständig regioselektiv.

### Schritt 1: Reduktion / katalytische Hydrierung

Die reduktive Dehalogenierung mittels katalytischer Hydrierung ist für 5-Halo-1-alkyl-3-fluoralkyl-1H-pyrazol-4-carbaldehyde nicht bekannt. Hierbei könnte man erwarten, dass unter den Reaktionsbedingungen auch die Aldehydgruppe zumindest teilweise reagieren wird. Überraschenderweise reagieren die Pyrazolderivate der Formel (II) unter Reaktionsbedingungen selektiv zu Pyrazolderivaten der Formel (III).

Es ist als überraschend anzusehen, dass die reduktive Dehalogenierung von 5-Halo-1-alkyl-3-fluoralkyl-1H-pyrazol-4-carbaldehyden selektiv und in hoher Ausbeute zu den 1-Alkyl-3-fluoralkyl-1H-pyrazol-4-carbaldehyden führt.

Die Reaktionstemperaturen können bei der Durchführung des erfindungsgemäßen Verfahrens in einem größeren Bereich variiert werden. Im Allgemeinen arbeitet man bei Temperaturen von 0 °C bis 180°C, bevorzugt bei Temperaturen von 0°C bis 100°C und besonders bevorzugt bei Temperaturen von 20°C bis 80°C.

Die Reaktionszeit kann in Abhängigkeit von der Reaktivität der Edukte bis zu 20 Stunden betragen, wobei der Abbruch der Reaktion bei vollständigem Umsatz auch schon früher erfolgen kann. Bevorzugt sind Reaktionszeiten von 3-10 Stunden.

Die Reaktion wird in Gegenwart von Wasserstoff durchgeführt. Es können dabei sowohl reiner Wasserstoff als auch Mischungen aus Wasserstoff und einem inertem Gas (bis 1:1), wie zum Beispiel Stickstoff oder Argon eingesetzt werden. Die Reaktion wird bei Drücken von 1 bar bis 50 bar, bevorzugt 1 bar bis 20 bar und besonders bevorzugt 2 bar 10 bar durchgeführt.

Zum Abfangen des bei der Reaktion gebildeten Chlor- Brom- oder Jodwasserstoffs wird eine Base zugesetzt. Bei der zugesetzten Base kann sowohl eine anorganische Base wie Natriumcarbonat, Kaliumcarbonat, Natriumbicarbonat, Kaliumbicarbonat, Mono-, Di- oder Trinatrium- oder Trikaliumphosphat, Natriumhydroxid oder Kalimhydroxid oder eine organische Base wie Triethylamin, Tributylamin, Diazabicyclo-undecen (DBU), Diazabicyclononen (DBN), Pyridin, Lutidin, 2-,3- oder 4-Picolin oder Diazabicyclooctan (DABCO) verwendet werden. Bevorzugt wird Triethylamin verwendet. Es werden 0,5 bis 20 Moläquivalente, bevorzugt 0,5 bis 5 Moläquivalente und besonders bevorzugt 1 bis 5 Moläquivalente der Base bezogen auf das Substrat zugesetzt.

Für die katalytische Hydrierung zur Reduktion der Verbindung der allgemeinen Formel (II) kann als Katalysator ein beliebiger Hydrierkatalysator verwendet werden. Geeignete Katalysatoren enthalten gegebenenfalls ein oder mehrere Metalle der Gruppen 8 - 10 des Periodensystems auf einem beliebigen üblichen anorganischen Träger. In Frage kommen beispielsweise Edelmetallkatalysatoren, wie Rutheniumkatalysatoren, Palladiumkatalysatoren, Platinkatalysatoren und Rhodiumkatalysatoren, Raney-NickelKatalysatoren und Raney-Cobalt und Lindlar-Katalysatoren. Neben diesen heterogenen Katalysatoren können jedoch auch Hydrierungen an homogenen Katalysatoren durchgeführt werden, beispielsweise an dem Wilkinson-Katalysator. Die entsprechenden Katalysatoren können in geträgerter Form, beispielsweise auf Kohlenstoff (Kohle oder Aktivkohle), Aluminiumoxid, Siliciumdioxid, Zirkondioxid, Calciumcarbonat oder Titandioxid, verwendet werden. Entsprechende Katalysatoren sind dem Fachmann an sich bekannt. Insbesondere bevorzugt sind Palladium-Katalysatoren geträgert auf Calciumcarbonat. Die Katalysatoren können sowohl in ihrer wasserfeuchten als auch trockenen Form eingesetzt werden. Der eingesetzte Katalysator wird bevorzugt für mehrere Umsetzungen wiederverwendet. Im erfindungsgemäßen Verfahren wird der Katalysator, bezogen auf das eingesetzte Halo-1-alkyl-3-fluoralkyl-1H-pyrazol-4-carbaldehyd der Formel (II), in einer Konzentration von etwa 0,01 bis etwa 30 Gew.-% verwendet. Bevorzugt wird der Katalysator in einer Konzentration von etwa 0,1 bis etwa 5 Gew.-% verwendet.

Die Reaktion wird in Gegenwart eines Lösungsmittels durchgeführt. Geeignete Lösungsmittel sind: Alkohole, Ethylacetat, Isopropylacetat, THF, Methyl-Tetrahydrofuran, Dioxan, Toluol, Hexan, Heptan, Pentan oder Petrolether. Besonders bevorzugt verwendet man Methanol, Ethanol, DMSO, Dimethylacetamid, DMF oder NMP.

### Schritt 2: Chlorierung

Die Reste R¹ und R² in Formel (III) haben die oben angegebenen Bedeutungen. Ganz besonders bevorzugt sind 1-Methyl-3-difluormethyl-1H-pyrazol-4-carbaldehyd (III-1) und 1-Methyl-3-trifluormethyl-1H-pyrazol-4-carbaldehyd (III-2), insbesondere die Verbindung (III-1).

Die Chlorierung von Pyrazolaldehyden zu Säurechloriden wurde in WO 2008/086962 beschrieben.

Man setzt üblicherweise den 1-Alkyl-3-fluoralkyl-1H-pyrazol-4-carbaldehyd der Formel (III) und das Chlorierungsmittel im Molverhältnis 1:3 bis 1:2, bevorzugt 1:1,4 bis 1:1 ein.

Als Chlorierungsmittel kann man Chlor, oder ein Chlor-abgebendes Agens einsetzen. Die Reaktion kann gegebenenfalls in Gegenwart eines inerten Verdünnungsgases wie z.B. Stickstoff, Kohlendioxyd oder Edelgase durchgeführt werden. Geeignet als Chlorierungsmittel, ohne Anspruch auf Vollständigkeit zu erheben, sind beispielsweise Cl₂, SO₂Cl₂, SOCl₂, N-Chlorsuccinimid oder ein Gemisch derselben. Man setzt bevorzugt Cl₂, SO₂Cl₂, oder ein Gemisch derselben als Chlorierungsmittel ein. Besonders bevorzugt ist die Verwendung von SO₂Cl₂ und Cl₂ als Chlorierungsmittel.

Die Umsetzung von 1-alkyl-3-fluoralkyl-1H-pyrazol-4-carbaldehyden der Formel (III) mit dem Chlorierungsmittel erfolgt üblicherweise in Gegenwart eines Verdünnungsmittels, das sich unter den vorherrschenden Reaktionsbedingungen inert verhält. Man kann als Verdünnungsmittel beispielsweise einfach oder mehrfach chlorierte aliphatische oder aromatische Kohlenwasserstoffe oder Gemischte derselben einsetzen. Beispiele für geeignete Verdünnungsmittel sind Chlorbenzol, Dichlorbenzole, Trichlorbenzole, Chlortoluole, Chlorbenzotrifluoride, Methylenchlorid, Dichlorethan, Chloroform, Tetrachlorkohlenstoff. Bevorzugte Verdünnungsmittel sind Chlorbenzol, 1,2-Dichlorbenzol, 1,3-Dichlorbenzol, 1,4-Dichlorbenzol, 1,2,3-Trichlorbenzol, 1,2,4-Trichlorbenzol, 4-Chlor-trifluormethylbenzol, 1,3,5-Trichlorbenzol, 2-Chlortoluol, 3-Chlortoluol, 4-Chlortoluol oder ein Gemisch derselben. Besonders bevorzugt verwendet man Chlorbenzol und Dichlorbenzol.

Man setzt das Verdünnungsmittel üblicherweise im Verhältnis 20 : 1 bis 1: 20 , bevorzugt 10: 1 bis 1: 10 bezogen auf den substituierten Aldehyd (III) ein.

Gemäß der vorliegenden Erfindung erfolgt die Chlorierung unter radikalischen Bedingungen. Voraussetzung dafür ist die Bildung von Chlorradikalen.

Es ist bekannt, dass organische Peroxide oder Azoverbindungen unter der Einwirkung von Wärme und/oder Licht in Radikale zerfallen, die die radikalische Chlorierung initiieren.

Beispiele, ohne Anspruch auf Vollständigkeit, geeigneter Peroxide und Azoverbindungen sind tert.-Butylhydroperoxyd, Dibenzoylperoxid, Di(4-tert-butylcyclohexyl)peroxydicarbonat, 2,2'-Azobis(isobutyronitril), Dimethyl-2,2' -azobis(isobutyrat), 2,2' -Azobis(2,4-dimethylvaleronitril), Di-(2-ethylhexyl)-peroxydicarbonat, tert-Butylperoxypivalat, tert.-Amylperoxy-2-ethylhexanoat, tert-Butylperoxy-2-ethylhexanoat.

Bevorzugt setzt man folgende Radikalstarter ein: 2,2'-Azobis(isobutyronitril), 2,2'-Azobis(2,4-dimethylvaleronitril), Di(2-ethylhexyl)peroxydicarbonat, tert-Amylperoxy-2-ethylhexanoat, tert-Butyl peroxy-2-ethylhexanoat. Man setzt den Radikalstarter üblicherweise in einer Menge von 0,01 bis 1 Mol-%, vorzugsweise 0,1 bis 0,5 Mol-% bezogen auf den Aldehyd der Formel (III) ein.

Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Anstelle der Chlorierung können die 1-alkyl-3-fluoralkyl-1H-pyrazol-4-carbaldehyde der Formel (III) alternativ durch Umsetzung mit einer Perhalosäure (z.B. Periodsäure) in Gegenwart eines Verdünnungsmittels (z.B. Acetonitril) und in Gegenwart eines Oxidationsmittels (z.B. Pyridiniumchlorochromat (PCC)) in die 1-alkyl-3-fluoralkyl-1H-pyrazol-4-carbonsäuren der Formel (IV) 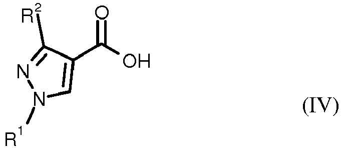 in welcher R¹ und R² die oben angegebenen Bedeutungen haben, umgesetzt werden.

Sowohl die 1-alkyl-3-fluoralkyl-1H-pyrazol-4-carbaldehyde der Formel (III) als auch die 1-alkyl-3-fluoralkyl-1H-pyrazol-4-carbonsäuren der Formel (IV) sind wichtige Zwischenstufen in der Synthese von Pflanzenschutzmitteln (vgl. z.B. WO 2007/087906).

Das erfindungsgemäße Herstellen von 1,3-dialkyl-1H-pyrazol-4-carbonsäurechloriden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiele

### Beispiel 1:

### 1-Methyl-3-difluormethyl-1H-pyrazol-4-carbaldehyd (III-1)

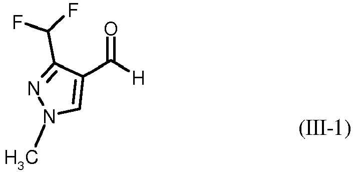

In einem Autoklaven wurden 10 g 5-Chlor-3-(difluormethyl)-1-methyl-1H-pyrazol-4-carbaldehyd in 150 mL Ethanol gelöst und 10,4 g Triethylamin sowie 500 mg 5 % Palladium auf Calciumcarbonat hinzugegeben. Der Autoklav wurde mit Stickstoff gespült und anschließend wurden 5 bar Wasserstoff aufgedrückt. Die Reaktionsmischung wurde 4 h bei 30 °C nachgerührt. Nach Filtration des Katalysators wurde das Lösungsmittel im Vakuum entfernt und das Produkt als Feststoff (7.4 g) mit einem Schmelzpunkt von 46-47°C erhalten.
¹H NMR (CDCl₃) 4,1 (s, 3H), 6,85 (t, 1H), 7,73 (s, 1H), 10, 1 (s, 1H) ppm.

### Beispiel 2:

### Ethyl-4,4-difluor-3-oxobutanoat (VI-1)

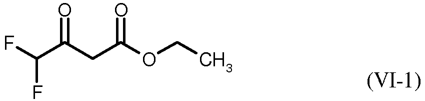

Unter Stickstoffatmosphäre wurden 46,7 g (1,168 mol) Natriumhydrid (60 % Dispersion in Parafin) zu 600 ml Tetrahydrofuran gegeben. Bei 35°C ließ man ein Gemisch aus 125 g (1,008 mol) Difluorethylacetat und 88,7 g (1,010 mol) Ethylacetat zutropfen, wobei die Temperatur unter 40°C gehalten wurde. Anschließend wurde bei Raumtemperatur über Nacht weiter gerührt. Das Reaktionsgemisch wurde vorsichtig in 1,7 L Eiswasser geschüttet und der pH-Wert durch Zugabe von Schwefelsäure auf pH 3 eingestellt. Man extrahierte zweimal mit je 500 ml Methyl-tert-butyl-ether, wusch die vereinigten organischen Phasen zweimal mit gesättigter Natriumchloridlösung, trocknete über Natriumsulfat, engte bei 40°C und 150 mbar ein und destillierte bei 60 mbar (Vigreux-Kolonne). Das Produkt wurde bei 85-87°C als farblose Flüssigkeit erhalten (104 g, 62 % der Theorie mit einer Reinheit von > 99 % (GC)).

### Beispiel 3:

### 3-(Difluormethyl)-1-methyl-1H-pyrazol-5-ol (VIII-1)

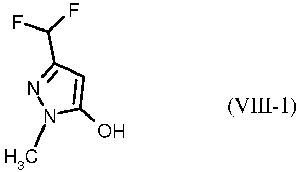

166 g (1 Mol) von Ethyl-4,4-difluor-3-oxobutanoat wurden in 500 ml Methyltert-butylether vorgelegt und mit 140 g Ameisensäure versetzt. Nach dem Abkühlen des Gemisches auf 5°C wurden 119 g (als 40 % wässrige Lösung) von Monomethylhydrazin zugegeben. Das Gemisch wurde 20 Std bei 22°C nachgerührt. Die Phasen wurden getrennt, die organische Phase mit 200 ml Wasser gewaschen und mit MgSO₄ getrocknet. Nach den Entfernen des Lösungsmittels im Vakuum erhielt man 148 g des Produktes als gelben Feststoff mit einem Scmp. Von 133°C und einer Reinheit von 95 %. Ausbeute 95 %.

### Beispiel 4:

### 5-Chlor-3-(difluormethyl)-1-methyl-1H-pyrazol-4-carbaldehyd (II-1)

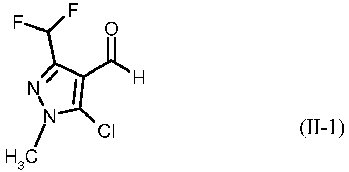

Zu einer Lösung aus 137,5 g (929 mmol) 3-(Difluormethyl)-1-methyl-1H-pyrazol-5-ol in 136 ml (1858 mmol) Dimethylformamid und 750 ml Toluol ließ man unter Kühlung 571 g (3716 mmol) Phosphoroxychlorid zutropfen. Man erhitzte das Gemisch für 3 Stunden unter Rückfluss, ließ abkühlen und goss das Reaktionsgemisch vorsichtig in 4 L Eiswasser. Man extrahierte das Produkt dreimal mit je 1500 mL Ethylacetat, wusch die vereinigten organischen Phasen zweimal mit gesättigter Natriumhydrogencarbonatlösung und schließlich mit gesättigter Natriumchloridlösung, trocknete über Natriumsulfat und engte ein. Man erhielt 138 g ( mit einer Reinheit von > 99 % (GC)) an 5-Chlor-3-(difluormethyl)-1-methyl-1H-pyrazol-4-carbaldehyd (II-1) in Form eines gelben-braunen Feststoffs, welcher ohne weitere Reinigung im nächsten Reaktionsschritt eingesetzt wurde.

### Beispiel 5:

### 1-Methyl-3-difluormethyl-1H-pyrazol-4-carbonsäurechlorid

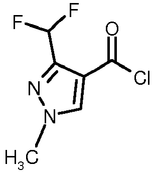

Die Lösung von 16 g (100 mol) von 1-Methyl-3-difluormethyl-1H-pyrazol-4-carbaldehyd, 13,5 g (100 mmol) Sulfurylchlorid und 0,2 g 2,2,-Azoisobutyronitril in 50 ml Chlorbenzol wurde bei 70-80°C für 6 Std. gerührt. Die Reaktionslösung wurde eingeengt. Man erhielt 18,8 g des Produktes (95 % Ausbeute) als Öl mit der Reinheit (GC) von 98 %.

## Patentansprüche

1. Verfahren zum Herstellen von 1-alkyl-3-fluoralkyl-1H-pyrazol-4-carbonsäurechloriden der Formel (I) 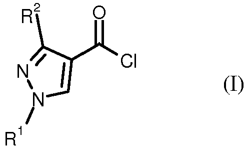 in welcher R¹ für C₁-C₆-Alkyl und R² für C₁-C₅-Fluoralkyl stehen, **dadurch gekennzeichnet, dass** man 5-Halo-1-alkyl-3-fluoralkyl-1H-pyrazol-4-carbaldehyde der Formel (II) 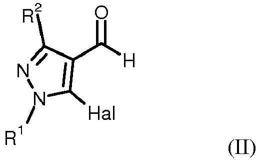 in welcher R¹ und R² die oben angegebenen Bedeutungen haben und Hal für Cl, Br oder I steht, in einem ersten Schritt mittels katalytischer Hydrierung und unter Zusatz einer Base zu 1-alkyl-3-fluoralkyl-1H-pyrazol-4-carbaldehyd der Formel (III) 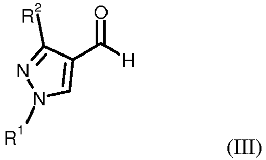 in welcher R¹ und R² die oben angegebenen Bedeutungen haben, umsetzt,
und dann die Verbindungen der Formel (III) durch Umsetzung mit einem Chlorierungsmittel unter Zusatz eines Radikalstarters in die Säurechloride der Formel (I) überführt.

2. Verfahren gemäß Anspruch 1, **dadurch gekennzeichnet, dass** man 5-Halo-1-alkyl-3-fluoralkyl-1H-pyrazol-4-carbaldehyde der Formel (II) einsetzt, in welchen R¹ für Methyl, Ethyl, n-Propyl, Isopropyl, Butyl, Pentyl, R² für CF₂H, CF₃, CF₂Cl, CCl₂F, C₂F₅, C₃F₇ und Hal für Cl stehen.

3. Verfahren gemäß Anspruch 1 oder 2, **dadurch gekennzeichnet, dass** man 5-Halo-1-alkyl-3-fluoralkyl-1H-pyrazol-4-carbaldehyde der Formel (II) einsetzt, in welchen R¹ für Methyl, R²- für CF₂H oder CF₃ und Hal für Cl stehen.

4. Verfahren gemäß einem der Ansprüche 1 bis 3, **dadurch gekennzeichnet, dass** man 5-Chlor-1-methyl-3-difluormethyl-1H-pyrazol-4-carbaldehyd (II-1) einsetzt.

5. Verfahren gemäß einem der Ansprüche 1 bis 4, **dadurch gekennzeichnet, dass** die katalytische Hydrierung unter Verwendung eines auf Calciumcarbonat geträgerten Palladium-Katalysators durchgeführt wird.

6. Verfahren gemäß einem der Ansprüche 1 bis 5, **dadurch gekennzeichnet, dass** die katalytische Hydrierung bei einer Temperatur von 0°C bis 100°C durchgeführt wird.

7. Verfahren gemäß einem der Ansprüche 1 bis 6, **dadurch gekennzeichnet, dass** die katalytische Hydrierung bei einem Druck von 1 bis 20 bar durchgeführt wird.

8. Verfahren gemäß einem der Ansprüche 1 bis 7, **dadurch gekennzeichnet, dass** die bei der katalytischen Hydrierung zugesetzte Base Triethylamin ist.

9. Verfahren gemäß einem der Ansprüche 1 bis 8, **dadurch gekennzeichnet, dass** bei der katalytischen Hydrierung 0,5 bis 5 Moläquivalente Base bezogen auf die Verbindung der Formel (II) zugesetzt wird.

10. Verfahren gemäß einem der Ansprüche 1 bis 9, **dadurch gekennzeichnet, dass** als Chlorierungsmittel SO₂Cl₂ verwendet wird.

11. Verfahren gemäß einem der Ansprüche 1 bis 10, **dadurch gekennzeichnet, dass** der Radikalstarter ausgewählt ist aus 2,2'-Azobis(isobutyronitril), 2,2'-Azobis(2,4-dimethylvaleronitril), Di(2-ethylhexyl)peroxydicarbonat, tert-Amylperoxy-2-ethylhexanoat und tert-Butyl peroxy-2-ethylhexanoat.

12. Verfahren gemäß einem der Ansprüche 1 bis 11, **dadurch gekennzeichnet, dass** der Radikalstarter in einer Menge von 0,1 bis 0,5 Mol-% bezogen auf das 1-alkyl-3-fluoralkyl-1H-pyrazol-4-carbaldehyd der Formel (III) eingesetzt wird.

## Claims

1. Method for preparing 1-alkyl-3-fluoroalkyl-1H-pyrazole-4-carbonyl chlorides of the formula (I) 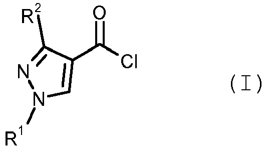 where R¹ is C₁-C₆-alkyl and R² is C₁-C₅-fluoroalkyl, **characterised in that** 5-halo-l-alkyl-3-fluoroalkyl-1H-pyrazole-4-carbaldehyde of the formula (II) 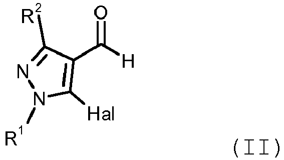 where R¹ and R² have the meanings stated above and Hal is Cl, Br or I,
is reacted in a first step by means of catalytic hydrogenation and with addition of a base to give
1-alkyl-3-fluoroalkyl-1H-pyrazole-4-carbaldehyde of the formula (III) 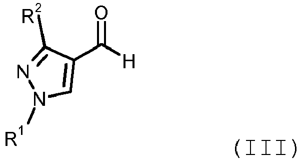 where R¹ and R² have the meanings stated above,
and then the compounds of the formula (III) are converted into the acyl chlorides of the formula (I) by reaction with a chlorinating agent with addition of a free-radical initiator.

2. Method according to Claim 1, **characterised in that** 5-halo-1-alkyl-3-fluoroalkyl-1H-pyrazole-4-carbaldehydes of the formula (II) where R¹ is methyl, ethyl, *n*-propyl, isopropyl, butyl, pentyl, R² is CF₂H, CF₃, CF₂Cl, CCl₂F, C₂F₅, C₃F₇ and Hal is Cl are used.

3. Method according to Claim 1 or 2, **characterised in that** 5-halo-1-alkyl-3-fluoroalkyl-1H-pyrazole-4-carbaldehydes of the formula (II) where R¹ is methyl, R² is CF₂H or CF₃ and Hal is Cl are used.

4. Method according to any of Claims 1 to 3, **characterised in that** 5-chloro-1-methyl-3-difluoromethyl-1H-pyrazole-4-carbaldehyde (II-1) is used.

5. Method according to any of Claims 1 to 4, **characterised in that** the catalytic hydrogenation is carried out using a palladium catalyst supported on calcium carbonate.

6. Method according to any of Claims 1 to 5, **characterised in that** the catalytic hydrogenation is carried out at a temperature of 0°C to 100°C.

7. Method according to any of Claims 1 to 6, **characterised in that** the catalytic hydrogenation is carried out at a pressure of 1 to 20 bar.

8. Method according to any of Claims 1 to 7, **characterised in that** the base added to the catalytic hydrogenation is triethylamine.

9. Method according to any of Claims 1 to 8, **characterised in that** 0.5 to 5 molar equivalents of base, based on the compound of the formula (II), is added in the catalytic hydrogenation.

10. Method according to any of Claims 1 to 9, **characterised in that** SO₂Cl₂ is used as chlorinating agent.

11. Method according to any of Claims 1 to 10, **characterised in that** the free-radical initiator is selected from the group consisting of 2,2'-azobis(isobutyronitrile), 2,2'-azobis(2,4-dimethylvaleronitrile), di(2-ethylhexyl) peroxydicarbonate, *tert*-amyl peroxy-2-ethylhexanoate and *tert*-butyl peroxy-2-ethylhexanoate.

12. Method according to any of Claims 1 to 11, **characterised in that** the free-radical initiator is used in an amount from 0.1 to 0.5 mol%, based on the 1-alkyl-3-fluoroalkyl-1H-pyrazole-4-carbaldehyde of the formula (III).

## Revendications

1. Procédé pour la préparation de chlorures de l'acide 1-alkyl-3-fluoroalkyl-1H-pyrazole-4-carboxylique de formule (I) 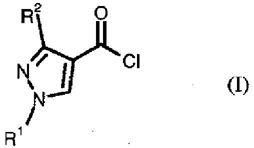 dans laquelle R¹ représente C₁-C₆-alkyle et R² représente C₁-C₅-fluoroalkyle, **caractérisé en ce qu'**on transforme des 5-halogéno-1-alkyl-3-fluoroalkyl-1H-pyrazole-4-carbaldéhydes de formule (II) 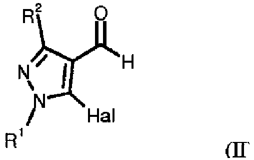 dans laquelle R¹ et R² présentent la signification susmentionnée et Hal représente Cl, Br ou I, dans une première étape, au moyen d'une hydrogénation catalytique et avec addition d'une base, en 1-alkyl-3-fluoroalkyl-1H-pyrazole-4-carbaldéhyde de formule (III) 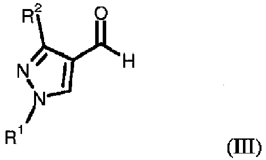 dans laquelle R¹ et R² présentent les significations susmentionnées, puis on transforme les composés de formule (III) par transformation avec un agent de chloration, avec addition d'un initiateur radicalaire, en chlorures d'acide de formule (I).

2. Procédé selon la revendication 1, **caractérisé en ce qu'**on utilise des 5-halogéno-1-alkyl-3-fluoroalkyl-1H-pyrazole-4-carbaldéhydes de formule (II) dans laquelle R¹ représente méthyle, éthyle, n-propyle, isopropyle, butyle, pentyle, R² représente CF₂H, CF₃, CF₂Cl, CCl₂F, C₂F₅, C₃F₇ et Hal représente Cl.

3. Procédé selon la revendication 1 ou 2, **caractérisé en ce qu'**on utilise des 5-halogéno-1-alkyl-3-fluoroalkyl-1H-pyrazole-4-carbaldéhydes de formule (II) dans laquelle R¹ représente méthyle, R² représente CF₂H ou CF₃ et Hal représente Cl.

4. Procédé selon l'une quelconque des revendications 1 à 3, **caractérisé en ce qu'**on utilise du 5-chloro-1-méthyl-3-difluorométhyl-1H-pyrazole-4-carbaldéhyde (II-1).

5. Procédé selon l'une quelconque des revendications 1 à 4, **caractérisé en ce que** l'hydrogénation catalytique est réalisée en utilisant un catalyseur de type palladium supporté sur du carbonate de calcium.

6. Procédé selon l'une quelconque des revendications 1 à 5, **caractérisé en ce que** l'hydrogénation catalytique est réalisée à une température de 0 à 100°C.

7. Procédé selon l'une quelconque des revendications 1 à 6, **caractérisé en ce que** l'hydrogénation catalytique est réalisée à une pression de 1 à 20 bars.

8. Procédé selon l'une quelconque des revendications 1 à 7, **caractérisé en ce que** la base ajoutée lors de l'hydrogénation catalytique est la triéthylamine.

9. Procédé selon l'une quelconque des revendications 1 à 8, **caractérisé en ce qu'**on ajoute 0,5 à 5 équivalents en mole de base, par rapport au composé de formule (I), lors de l'hydrogénation catalytique.

10. Procédé selon l'une quelconque des revendications 1 à 9, **caractérisé en ce qu'**on utilise du SO₂Cl₂ comme agent de chloration.

11. Procédé selon l'une quelconque des revendications 1 à 10, **caractérisé en ce que** l'initiateur radicalaire est choisi parmi le 2,2'-azobis(isobutyronitrile), le 2,2'-azobis(2,4-diméthylvaléronitrile), le peroxydicarbonate de di(2-éthylhexyle), le peroxy-2-éthylhexanoate de tert-amyle et le peroxy-2-éthylhexanoate de tert-butyle.

12. Procédé selon l'une quelconque des revendications 1 à 11, **caractérisé en ce que** l'initiateur radicalaire est utilisé en une quantité de 0,1 à 0,5% en mole par rapport au 1-alkyl-3-fluoroalkyl-1H-pyrazole-4-carbaldéhyde de formule (III) .
